# EUROPEAN PATENT APPLICATION

(11) **EP 4 734 052 A1**
(43) Date of publication of application: **29.04.2026**
(21) Application number: 25211101.8
(22) Date of filing: 24.10.2025
(51) Int. Cl.: G06T 7/11

(54) **SYSTEM FOR TRAINING AND UTILIZING A SUBJECT SPECIFIC CONVOLUTIONAL NEURAL NETWORK (CNN) IN LONGITUDINAL SCANS**

(30) Priority: 25.10.2024 US 202418927298
(71) Applicant: Springbok, Inc., Charlottesville, Virginia 22902 (US)
(72) Inventor: RIEM, Lara, Charlottesville (US); FENG, Xue, Charlottesville (US); BLEMKER, Silvia, Charlottesville (US)
(74) Representative: Maiwald GmbH

(57) **Abstract**

The present disclosure relates to systems and methods for segmenting one or more regions of interest in medical image data. These include inputting a plurality of medical images into each of a plurality of convolutional neural networks (CNNs); segmenting the plurality of medical images using the plurality of CNNs; identifying a group of a plurality of voxels belonging to one or more ROI in the segmented plurality of medical images; calculating a plurality of variables from the segmented plurality of medical images; and outputting at least one of segmented ROI or a change in the plurality of variables.

## Description

### Cross Reference to Related Applications

N/A

### Statement of Government Support

This invention was made with government support under R44AR078720 awarded by the National Institute of Health. The government has certain rights in the invention.

### Background

Image analysis of the musculoskeletal system is often performed manually, which is time consuming. Moreover, manual analysis leads to subjective results that vary based on who performs the analysis, the processing and visualization tools available, and imaging protocols used. Several techniques have been developed for automatic muscle segmentation, such as atlas-based and shape-based methods. Recently, deep convolutional neural network (DCNN) has been applied in many medical image segmentation tasks from MRI or computed tomography (CT) images.

However, these CNNs perform at varying levels depending on the muscle or region of interest being segmented. Furthermore, these CNNs underperform when applied to longitudinal studies. For example, these suboptimal CNNs can make mistakes when analyzing changes in muscle within a single subject over time.

Thus, there is a need to improve subject-specific segmentation accuracy of CNN outputs for longitudinal and disease progression studies.

### Summary

The present disclosure provides systems and methods that overcome the aforementioned drawbacks by finetuning a multi-model system with vetted patient-specific data, to increase labeling accuracy of the patient over time.

In one aspect of the present disclosure, a system for segmenting one or more regions of interest (ROI) in medical image data is presented. The system comprises a memory configured to store instructions and a plurality of medical images of a subject at a first set of different time points. The system further includes a processor configured to access the memory, fine tune a plurality of trained convolutional neural networks (CNNs) using the first set of the plurality of segmented medical images, access a second set of a plurality of medical images of the subject, wherein the second set of the plurality of medical images comprise a plurality of pixels or voxels, segment the second set of the plurality of medical images by inputting the second set of the plurality of medical images into the plurality of finetuned CNNs, identify one or more groups of the plurality of pixels or voxels belonging to one or more ROI, calculate a plurality of variables from the segmented plurality of medical images, and output at least one of segmented ROI or a change in the plurality of variables. The system further comprises a display configured to display the output.

In another aspect of the present disclosure, a method of segmenting one or more regions of interest in medical image data is presented. The method comprises accessing a plurality of medical images of a subject at a first set of different time points using a processor, the medical images comprising a plurality of pixels or voxels, inputting the plurality of medical images into each of a plurality of convolutional neural networks (CNNs), segmenting the plurality of medical images using the plurality of CNNs, identifying a group of the plurality of pixels or voxels belonging to one or more regions of interest (ROI) in the segmented plurality of medical images, calculating a plurality of variables from the segmented plurality of medical images, and outputting at least one of segmented ROI or a change in the plurality of variables.

These aspects are nonlimiting. Other aspects and features of the systems and methods described herein will be provided below.

### Brief Description of the Drawings

The foregoing features of embodiments will be more readily understood by reference to the following detailed description, taken with reference to the accompanying drawings, in which:
FIG. 1 is a schematic diagram of one non-limiting example of a framework for implementing a multi-model platform in accordance with the present disclosure.
FIG. 2 is a block diagram of an example ROI label prediction system.
FIG. 3 is a block diagram of example components that can implement the ROI label prediction system of FIG. 2.
FIG. 4A is a flow chart setting forth some non-limiting example steps of a process that can be implemented using a platform, for example, such as described with respect to FIG. 1.
FIG. 4B is a flow chart setting forth some non-limiting example steps of an implementation using a platform.

### Detailed Description

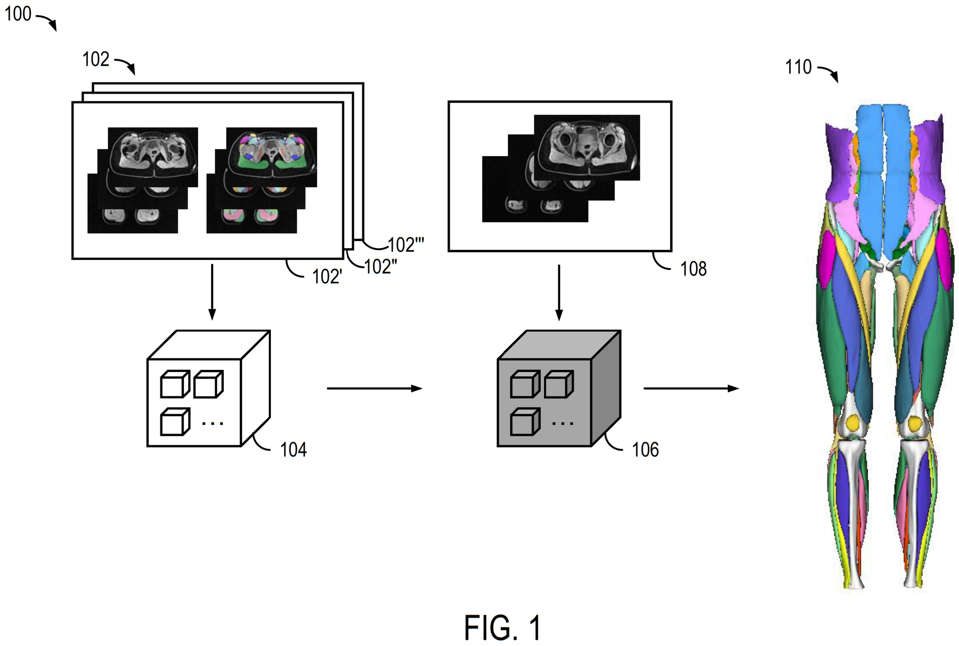

Referring now to Fig. 1, a schematic diagram of a non-limiting example of a framework 100 for implementing a multi-modal AI platform. A first set of a plurality of medical images 102 for a patient at one or more different time points 102', 102", 102"' are deployed on a plurality of previously trained convolutional neural networks (CNNs) 104 to fine tune the plurality of CNNs 104. As used herein, "fine tune" refers to loading in the previously trained CNNs and performing additional training rounds. In a non-limiting example, the previously trained CNNs are finetuned by modifying the learning rate of the models once loaded in or increasing the number of epochs the models are trained on. By increasing the learning weight, the training inputs (i.e., the first set of a plurality of medical images 102) from an individual are weighted higher than the previous training data. In a non-limiting example, the plurality of medical images 102 include segmented regions of interest (ROI). For example, the ROI may include, but is not limited to, one or more muscles. In a non-limiting example, the medical images include magnetic resonance (MR) images. The schematic of FIG. 1 illustrates MR images but is not intended to be limiting. Further, the plurality of trained CNNs 104 differ in one or more parameters. For example, a parameter may include a network structure, one or more parameters during training, a training set, or one or more parameters during deployment of the system. In a non-limiting example, the parameter may include a window size, learning rate, training input resolution, training dataset, or whether the training data is augmented.

In a non-limiting example, the resulting plurality of fine-tuned CNNs 106 may be stored until a second set of a plurality of medical images 108 are deployed on the fine-tuned CNNs 106. The second set of a plurality of medical images 108 are distinct from the first set of a plurality of medical images 102, and thus were not used to fine tune the trained CNNs 104. Further, the second set of a plurality of medical images are not segmented or labeled or otherwise annotated with respect to one or more ROI. In a non-limiting example, the second set of a plurality of medical images 108 may be, but are not limited to, MR images. In a non-limiting example, the second set of a plurality of medical images are a more recent set of medical images taken of a subject, than the first set of a plurality of medical images 102.

The fine-tuned CNNs 106 deployed with the second set of a plurality of medical images 108 output three dimensional (3D) segmented ROIs 110 that may be presented to a user on a display.

As used herein, the term "CNN" may be interchangeable with "model" or "AI."

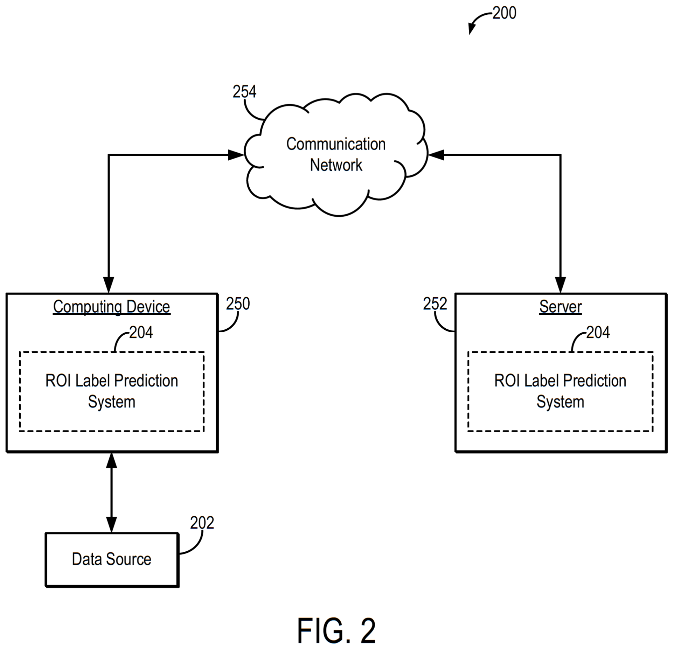

Referring now to FIG. 2, an example of a system 200 for generating ROI labels in accordance with some embodiments of the systems and methods described in the present disclosure is shown. As shown in FIG. 2, a computing device 250 can receive one or more types of medical image data (e.g., magnetic resonance (MR) images) from data source 202. In some embodiments, computing device 250 can execute at least a portion of a ROI label prediction system 204 to generate ROI labels from data received from the data source 202.

Additionally or alternatively, in some embodiments, the computing device 250 can communicate information about data received from the data source 202 to a server 252 over a communication network 254, which can execute at least a portion of the ROI label prediction system 204. In such embodiments, the server 252 can return information to the computing device 250 (and/or any other suitable computing device) indicative of an output of the ROI label prediction system 204.

In some embodiments, computing device 250 and/or server 252 can be any suitable computing device or combination of devices, such as a desktop computer, a laptop computer, a smartphone, a tablet computer, a wearable computer, a server computer, a virtual machine being executed by a physical computing device, and so on. The computing device 2550 and/or server 252 can also reconstruct images from the data.

In some embodiments, data source 202 can be any suitable source of data (e.g., measurement data, images reconstructed from measurement data, processed image data), such as an MR system or another computing device (e.g., a server storing measurement data, images reconstructed from measurement data, processed image data), and so on. In some embodiments, data source 202 can be local to computing device 250. For example, data source 202 can be incorporated with computing device 250 (e.g., computing device 250 can be configured as part of a device for measuring, recording, estimating, acquiring, or otherwise collecting or storing data). As another example, data source 202 can be connected to computing device 250 by a cable, a direct wireless link, and so on. Additionally or alternatively, in some embodiments, data source 202 can be located locally and/or remotely from computing device 250, and can communicate data to computing device 250 (and/or server 252) via a communication network (e.g., communication network 254).

In some embodiments, communication network 254 can be any suitable communication network or combination of communication networks. For example, communication network 254 can include a Wi-Fi network (which can include one or more wireless routers, one or more switches, etc.), a peer-to-peer network (e.g., a Bluetooth network), a cellular network (e.g., a 3G network, a 4G network, etc., complying with any suitable standard, such as CDMA, GSM, LTE, LTE Advanced, WiMAX, etc.), other types of wireless network, a wired network, and so on. In some embodiments, communication network 254 can be a local area network, a wide area network, a public network (e.g., the Internet), a private or semi-private network (e.g., a corporate or university intranet), any other suitable type of network, or any suitable combination of networks. Communications links shown in FIG. 2 can each be any suitable communications link or combination of communications links, such as wired links, fiber optic links, Wi-Fi links, Bluetooth links, cellular links, and so on.

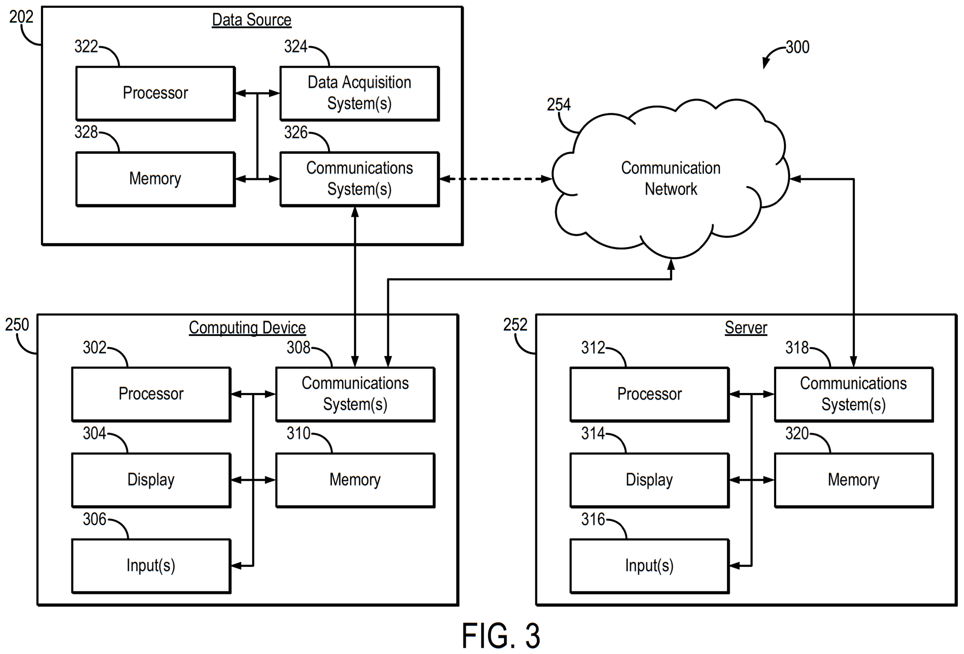

Referring now to FIG. 3, an example of hardware 300 that can be used to implement data source 202, computing device 250, and server 252 in accordance with some embodiments of the systems and methods described in the present disclosure is shown.

As shown in FIG. 3, in some embodiments, computing device 250 can include a processor 302, a display 304, one or more inputs 306, one or more communication systems 308, and/or memory 310. In some embodiments, processor 302 can be any suitable hardware processor or combination of processors, such as a central processing unit ("CPU"), a graphics processing unit ("GPU"), and so on. In some embodiments, display 304 can include any suitable display devices, such as a liquid crystal display ("LCD") screen, a light-emitting diode ("LED") display, an organic LED ("OLED") display, an electrophoretic display (e.g., an "e-ink" display), a computer monitor, a touchscreen, a television, and so on. In some embodiments, inputs 306 can include any suitable input devices and/or sensors that can be used to receive user input, such as a keyboard, a mouse, a touchscreen, a microphone, and so on.

In some embodiments, communications systems 308 can include any suitable hardware, firmware, and/or software for communicating information over communication network 254 and/or any other suitable communication networks. For example, communications systems 308 can include one or more transceivers, one or more communication chips and/or chip sets, and so on. In a more particular example, communications systems 308 can include hardware, firmware, and/or software that can be used to establish a Wi-Fi connection, a Bluetooth connection, a cellular connection, an Ethernet connection, and so on.

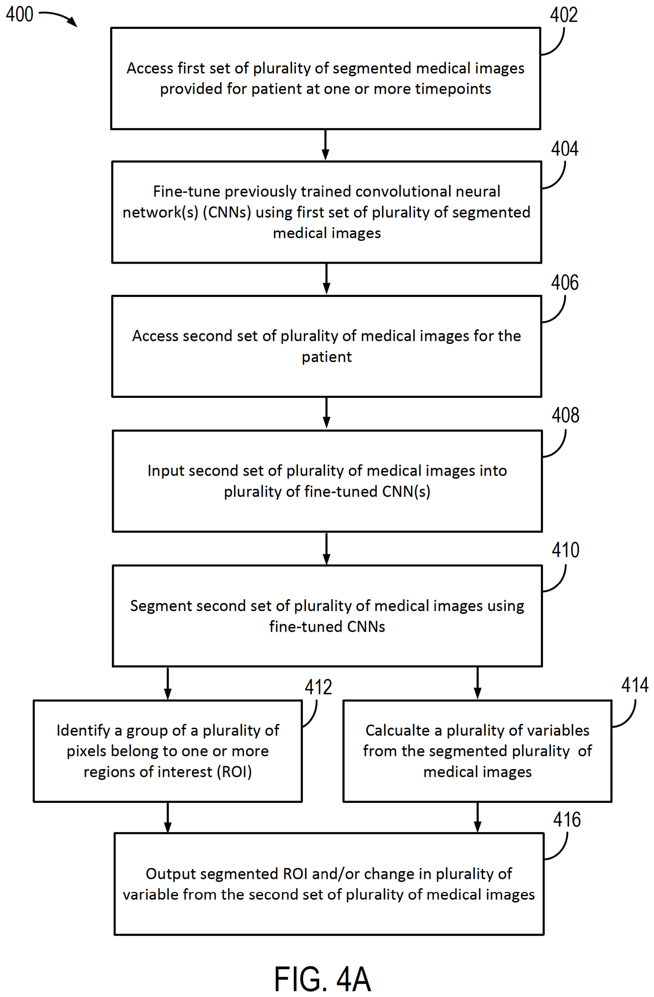

In some embodiments, memory 310 can include any suitable storage device or devices that can be used to store instructions, values, data, or the like, that can be used, for example, by processor 302 to present content using display 304, to communicate with server 252 via communications system(s) 308, and so on. Memory 310 can include any suitable volatile memory, non-volatile memory, storage, or any suitable combination thereof. For example, memory 310 can include random-access memory ("RAM"), read-only memory ("ROM"), electrically programmable ROM ("EPROM"), electrically erasable ROM ("EEPROM"), other forms of volatile memory, other forms of non-volatile memory, one or more forms of semi-volatile memory, one or more flash drives, one or more hard disks, one or more solid state drives, one or more optical drives, and so on. In some embodiments, memory 310 can have encoded thereon, or otherwise stored therein, a computer program for controlling operation of computing device 250. In such embodiments, processor 302 can execute at least a portion of the computer program to present content (e.g., images, user interfaces, graphics, tables), receive content from server 252, transmit information to server 252, and so on. For example, the processor 302 and the memory 310 can be configured to perform the methods described herein (e.g., the methods of FIGS. 4A-4B).

In some embodiments, server 252 can include a processor 312, a display 314, one or more inputs 316, one or more communications systems 318, and/or memory 320. In some embodiments, processor 312 can be any suitable hardware processor or combination of processors, such as a CPU, a GPU, and so on. In some embodiments, display 314 can include any suitable display devices, such as an LCD screen, LED display, OLED display, electrophoretic display, a computer monitor, a touchscreen, a television, and so on. In some embodiments, inputs 316 can include any suitable input devices and/or sensors that can be used to receive user input, such as a keyboard, a mouse, a touchscreen, a microphone, and so on.

In some embodiments, communications systems 318 can include any suitable hardware, firmware, and/or software for communicating information over communication network 254 and/or any other suitable communication networks. For example, communications systems 318 can include one or more transceivers, one or more communication chips and/or chip sets, and so on. In a more particular example, communications systems 318 can include hardware, firmware, and/or software that can be used to establish a Wi-Fi connection, a Bluetooth connection, a cellular connection, an Ethernet connection, and so on.

In some embodiments, memory 320 can include any suitable storage device or devices that can be used to store instructions, values, data, or the like, that can be used, for example, by processor 312 to present content using display 314, to communicate with one or more computing devices 250, and so on. Memory 320 can include any suitable volatile memory, non-volatile memory, storage, or any suitable combination thereof. For example, memory 320 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 320 can have encoded thereon a server program for controlling operation of server 252. In such embodiments, processor 312 can execute at least a portion of the server program to transmit information and/or content (e.g., data, images, a user interface) to one or more computing devices 250, receive information and/or content from one or more computing devices 250, receive instructions from one or more devices (e.g., a personal computer, a laptop computer, a tablet computer, a smartphone), and so on.

In some embodiments, the server 252 is configured to perform the methods described in the present disclosure. For example, the processor 312 and memory 320 can be configured to perform the methods described herein (e.g., the methods of FIGS. 4A-4B).

In some embodiments, data source 202 can include a processor 322, one or more data acquisition systems 324, one or more communications systems 326, and/or memory 328. In some embodiments, processor 322 can be any suitable hardware processor or combination of processors, such as a CPU, a GPU, and so on. In some embodiments, the one or more data acquisition systems 324 are generally configured to acquire data, images, or both, and can include medical imaging systems (e.g., MR system). Additionally or alternatively, in some embodiments, the one or more data acquisition systems 324 can include any suitable hardware, firmware, and/or software for coupling to and/or controlling operations of the medical imaging systems. In some embodiments, one or more portions of the data acquisition system(s) 324 can be removable and/or replaceable.

Note that, although not shown, data source 202 can include any suitable inputs and/or outputs. For example, data source 202 can include input devices and/or sensors that can be used to receive user input, such as a keyboard, a mouse, a touchscreen, a microphone, a trackpad, a trackball, and so on. As another example, data source 202 can include any suitable display devices, such as an LCD screen, an LED display, an OLED display, an electrophoretic display, a computer monitor, a touchscreen, a television, etc., one or more speakers, and so on.

In some embodiments, communications systems 326 can include any suitable hardware, firmware, and/or software for communicating information to computing device 250 (and, in some embodiments, over communication network 254 and/or any other suitable communication networks). For example, communications systems 326 can include one or more transceivers, one or more communication chips and/or chip sets, and so on. In a more particular example, communications systems 326 can include hardware, firmware, and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, and so on.

In some embodiments, memory 328 can include any suitable storage device or devices that can be used to store instructions, values, data, or the like, that can be used, for example, by processor 322 to control the one or more data acquisition systems 324, and/or receive data from the one or more data acquisition systems 324; to generate images from data; present content (e.g., data, images, a user interface) using a display; communicate with one or more computing devices 250; and so on. Memory 328 can include any suitable volatile memory, non-volatile memory, storage, or any suitable combination thereof. For example, memory 328 can include RAM, ROM, EPROM, EEPROM, other types of volatile memory, other types of non-volatile memory, one or more types of semi-volatile memory, one or more flash drives, one or more hard disks, one or more solid state drives, one or more optical drives, and so on. In some embodiments, memory 328 can have encoded thereon, or otherwise stored therein, a program for controlling operation of data source 202. In such embodiments, processor 322 can execute at least a portion of the program to generate images, transmit information and/or content (e.g., data, images, a user interface) to one or more computing devices 250, receive information and/or content from one or more computing devices 250, receive instructions from one or more devices (e.g., a personal computer, a laptop computer, a tablet computer, a smartphone, etc.), and so on.

In some embodiments, any suitable computer-readable media can be used for storing instructions for performing the functions and/or processes described herein. For example, in some embodiments, computer-readable media can be transitory or non-transitory. For example, non-transitory computer-readable media can include media such as magnetic media (e.g., hard disks, floppy disks), optical media (e.g., compact discs, digital video discs, Blu-ray discs), semiconductor media (e.g., RAM, flash memory, EPROM, EEPROM), any suitable media that is not fleeting or devoid of any semblance of permanence during transmission, and/or any suitable tangible media. As another example, transitory computer-readable media can include signals on networks, in wires, conductors, optical fibers, circuits, or any suitable media that is fleeting and devoid of any semblance of permanence during transmission, and/or any suitable intangible media.

As used herein in the context of computer implementation, unless otherwise specified or limited, the terms "component," "system," "module," "framework," and the like are intended to encompass part or all of computer-related systems that include hardware, software, a combination of hardware and software, or software in execution. For example, a component may be, but is not limited to being, a processor device, a process being executed (or executable) by a processor device, an object, an executable, a thread of execution, a computer program, or a computer. By way of illustration, both an application running on a computer and the computer can be a component. One or more components (or system, module, and so on) may reside within a process or thread of execution, may be localized on one computer, may be distributed between two or more computers or other processor devices, or may be included within another component (or system, module, and so on).

Referring now to FIG. 4A, a flowchart 400 is provided for one, non-limiting example process that can be carried out using a platform, for example, such as described with respect to Fig. 1. The flowchart encompasses a fine-tuning (steps 402 - 404) and application (steps 406-416) workflow. The process 400 begins at process block 402, whereby a first set of a plurality of segmented medical images for a patient at one or more time points is accessed, using a processor. The first set of a plurality of segmented medical images may be accessed from a memory or directly from an imaging system. The first set of a plurality of segmented medical images may be obtained from the patient at various time intervals, such as hours, weeks, months, or years.

At step 404, the first set of the plurality of segmented medical images are input into a plurality of trained CNNs for fine-tuning the plurality of trained CNNs. In a non-limiting example, each of the plurality of trained CNNs vary in at least one of a network structure, one or more network parameters during training, a training set, or one or more parameters during deployment. Further, the CNNs may be trained on a training set comprising segmented medical images from other subjects. Alternatively, the training set may include the patient's previous medical images such that the CNNs are weighted to be more subject-specific. As used herein, "subject" and "patient" may be used interchangeably. In another non-limiting example, training the plurality of CNNs includes adjusting at least one of a training iteration or learning rate.

In a non-limiting example, the new set of training data may be the first set of the plurality of segmented medical images of a subject at different time points. Alternatively, the new set of training data may be subject's specific data that may have been used to train the CNN before for additional training.

At step 406, a second set of a plurality of medical images for the patient. Further, the second set of the plurality of medical images comprise a plurality of pixels or voxels. In a non-limiting example, the second set of the plurality of medical images may be, but are not limited to, MR images. The second set of the plurality of medical images may be accessed from a memory or directly from an imaging system. In a non-limiting example, the second set of the plurality of medical images are acquired from the patient chronologically later than the first set of the plurality of segmented medical images. Further, the second set of the plurality of medical image are not segmented, labeled, otherwise annotated with regard to one or more ROI.

At step 408, the second set of the plurality of medical images are input into the plurality of fine-tune CNNs for segmentation at step 410. In a non-limiting example, the plurality of fine tuned CNNs use a sliding widow approach to segment the second set of the plurality of medical images.

Next, at step 412, one or more ROI are identified from the one or more groups of the plurality of pixels or voxels of the segmented plurality of medical images. At step 414, occurring before, simultaneously, or after step 412, a plurality of variables is calculated from the segmented plurality of medical images. In a non-limiting example, the plurality of variables includes at least one of a median, a standard deviation, a label volume variation, a label dice, or a label probability between each of the plurality of the fine-tuned CNNs.

At step 416, the processor outputs at least one of the segmented ROI from step 412 or a change in the plurality of variables. The output may be displayed on a display as described in relation to FIG. 3 In a non-limiting example, the output may include 3D segmented ROIs that may be presented to a user on a display.

In a non-limiting example, the steps beginning at 406 may be repeated for subsequent acquisition of medical images of the subject.

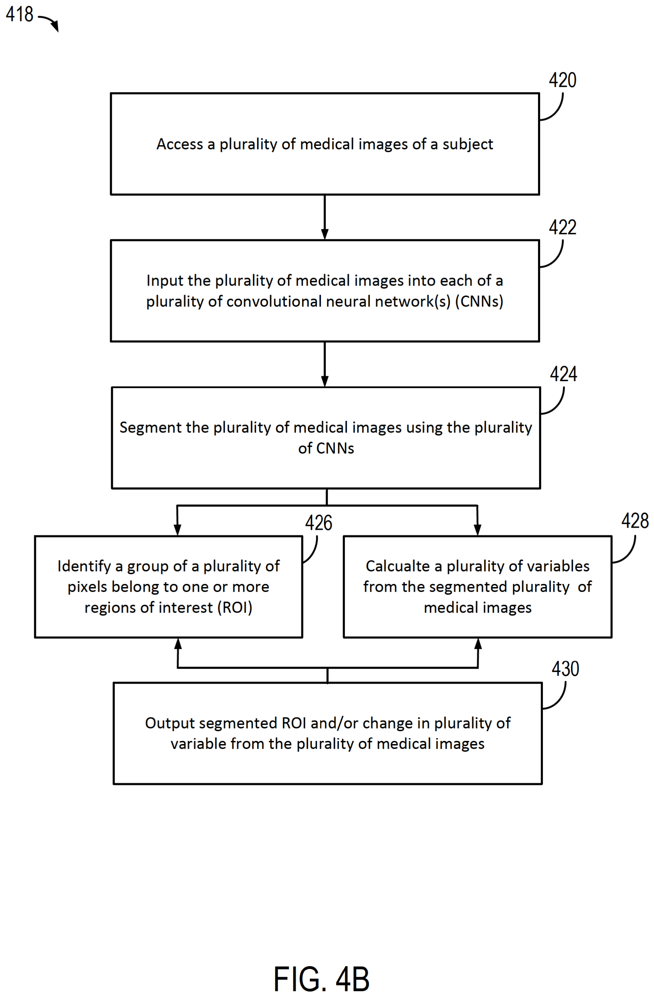

FIG. 4B represents a flowchart 418 of an application of using the plurality of fine-tuned CNNs described in FIGS. 1 and 4A to segment one or more ROI in medical image data of a subject. At step 420, a plurality of medical images of a subject is access by a processor. As described previously, the plurality of medical images comprises a plurality of pixels or voxels. In a non-limiting example, the second set of the plurality of medical images may be, but are not limited to, MR images. Further, the plurality of medical images may be accessed from a memory or directly from an imaging system. the plurality of medical image data is unsegmented, unlabeled, and otherwise not annotated regarding one or more ROI in the plurality of medical images.

Next, at step 422, the plurality of medical images is input into a plurality of CNNs and segmented at step 424. In a non-limiting example, the plurality of CNNs use a sliding widow approach to segment the second set of the plurality of medical images.

At step 426, one or more ROI are identified from the one or more groups of the plurality of pixels or voxels of the segmented plurality of medical images. At step 428, occurring before, simultaneously, or after step 426, a plurality of variables is calculated from the segmented plurality of medical images. In a non-limiting example, the plurality of variables includes at least one of a median, a standard deviation, a label volume variation, a label dice, or a label probability between each of the plurality of the CNNs.

At step 430, the processor outputs at least one of the segmented ROI from step 412 or a change in the plurality of variables. The output may be displayed on a display as described in relation to FIG. 3. In a non-limiting example, the output may include 3D segmented ROIs that may be presented to a user on a display.

As used in this specification and the claims, the singular forms "a," "an," and "the" include plural forms unless the context clearly dictates otherwise.

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean up to plus or minus 10% of the particular term and "substantially" and "significantly" will mean more than plus or minus 10% of the particular term.

As used herein, the terms "include" and "including" have the same meaning as the terms "comprise" and "comprising." The terms "comprise" and "comprising" should be interpreted as being "open" transitional terms that permit the inclusion of additional components further to those components recited in the claims. The terms "consist" and "consisting of" should be interpreted as being "closed" transitional terms that do not permit the inclusion of additional components other than the components recited in the claims. The term "consisting essentially of" should be interpreted to be partially closed and allowing the inclusion only of additional components that do not fundamentally alter the nature of the claimed subject matter.

The phrase "such as" should be interpreted as "for example, including." Moreover, the use of any and all exemplary language, including but not limited to "such as", is intended merely to better illuminate the invention and does not pose a limitation on the scope of the invention unless otherwise claimed.

Furthermore, in those instances where a convention analogous to "at least one of A, B and C, etc." is used, in general such a construction is intended in the sense of one having ordinary skill in the art would understand the convention (*e.g*., "a system having at least one of A, B and C" would include but not be limited to systems that have A alone, B alone, C alone, A and B together, A and C together, B and C together, and/or A, B, and C together.). It will be further understood by those within the art that virtually any disjunctive word and/or phrase presenting two or more alternative terms, whether in the description or figures, should be understood to contemplate the possibilities of including one of the terms, either of the terms, or both terms. For example, the phrase "A or B" will be understood to include the possibilities of "A" or "B" or "A and B."

All language such as "up to," "at least," "greater than," "less than," and the like, include the number recited and refer to ranges which can subsequently be broken down into ranges and subranges. A range includes each individual member. Thus, for example, a group having 1-3 members refers to groups having 1, 2, or 3 members. Similarly, a group having 6 members refers to groups having 1, 2, 3, 4, or 6 members, and so forth.

The modal verb "may" refers to the preferred use or selection of one or more options or choices among the several described embodiments or features contained within the same. Where no options or choices are disclosed regarding a particular embodiment or feature contained in the same, the modal verb "may" refers to an affirmative act regarding how to make or use an aspect of a described embodiment or feature contained in the same, or a definitive decision to use a specific skill regarding a described embodiment or feature contained in the same. In this latter context, the modal verb "may" has the same meaning and connotation as the auxiliary verb "can."

## Claims

1. A system for segmenting one or more regions of interest (ROI) in medical image data, comprising:
a memory configured to store instructions and a first set of a plurality of segmented medical images of a subject at different time points;
a processor configured to:
access the memory;
fine tune a plurality of trained convolutional neural networks (CNNs) using the first set of the plurality of segmented medical images;
access a second set of a plurality of medical images of the subject, wherein the second set of the plurality of medical images comprise a plurality of pixels or voxels;
segment the second set of the plurality of medical images by inputting the second set of the plurality of medical images into the plurality of finetuned CNNs;
identify one or more groups of the plurality of pixels or voxels belonging to one or more ROI;
calculate a plurality of variables from the segmented plurality of medical images;
output at least one of segmented ROI or a change in the plurality of variables; and a display configured to display the output.

2. The system of claim 1, wherein each of the plurality of trained CNNs vary in at least one of a network structure, one or more network parameters during training, a training set, or one or more parameters during deployment.

3. The system of claim 2, wherein the trained CNNs use a sliding window approach to segment the second set of the plurality of medical images.

4. The system of claim 1, wherein the plurality of variables includes at least one of a median, a standard deviation, a label volume variation, a label dice, or a label probability between each of the plurality of fine-tuned CNNs.

5. The system of claim 1, wherein each of the plurality of trained CNNs are trained using a training set of segmented medical images.

6. The system of claim 1, wherein the one or more ROI includes one or more muscles.

7. The system of claim 1, wherein the processor is further configured to generate a three-dimensional (3D) model that identifies the one or more groups of the plurality of pixels or voxels belonging to the one or more ROI of the subject.

8. The system of claim 1, wherein the first set of the plurality of segmented medical images and the second set of the plurality of medical images include magnetic resonance (MR) images.

9. The system of claim 1, wherein training the plurality trained CNNs includes adjusting at least one of a training iteration or a learning rate.

10. A method of segmenting one or more regions of interest in medical image data, comprising:
accessing a plurality of medical images of a subject at using a processor, the medical images comprising a plurality of pixels or voxels;
inputting the plurality of medical images into each of a plurality of convolutional neural networks (CNNs);
segmenting the plurality of medical images using the plurality of CNNs;
identifying a group of the plurality of pixels or voxels belonging to one or more regions of interest (ROI) in the segmented plurality of medical images;
calculating a plurality of variables from the segmented plurality of medical images; and
outputting at least one of segmented ROI or a change in the plurality of variables.

11. The method of claim 10, wherein the plurality of CNNs are trained using a training set of a plurality of medical images, and wherein the plurality of CNNS are finetuned using a set of a plurality of segmented medical images of the subject at different time points.

12. The method of claim 11, wherein training the plurality of CNNs further includes adjusting at least one of a training iteration or a learning rate.

13. The method of claim 10, wherein each of the plurality of CNNs vary in at least one of a network structure, one or more network parameters during training, a training set, or one or more parameters during deployment.

14. The method of claim 12, wherein the trained CNNs use a sliding window approach to segment the plurality of medical images.

15. The method of claim 10, wherein the plurality of variables includes at least one of a median, a standard deviation, a label volume variation, a label dice, or a label probability between each of the plurality of CNNs.

16. The method of claim 10, wherein the one or more ROI includes one or more muscles.

17. The method of claim 10, further comprising generating a three-dimensional (3D) model that identifies a group of the plurality of voxels belonging to one or more ROI of the subject.

18. The method of claim 10, wherein the plurality of medical images include magnetic resonance (MR) images.
